# EUROPEAN PATENT SPECIFICATION

(11) **EP 2 061 723 B1**
(45) Date of publication and mention of the grant of the patent: **18.11.2009**
(21) Application number: 07786841.2
(22) Date of filing: 26.06.2007
(51) Int. Cl.: C01G 45/00, A61Q 17/04, C09C 1/04

(54) **OXIDE PARTICLES COMPRISING ZINC AND MANGANESE**
ZINK UND MANGAN ENTHALTENDE OXIDPARTIKEL
PARTICULES D'OXYDE COMPRENANT DU ZINC ET DU MANGANÈSE

(30) Priority: 29.07.2006 DE 102006035136
(43) Date of publication of application: 27.05.2009
(73) Proprietor: Evonik Degussa GmbH, 45128 Essen (DE)
(72) Inventor: KATUSIC, Stipan, 65812 Bad Soden (DE); KRÖLL, Michael, 63589 Linsengericht (DE); HEBERER, Stefan, 63073 Offenbach (DE); KRESS, Peter, 63791 Karlstein (DE); ZIMMERMANN, Guido, 50321 Brühl (DE)
(86) International application number: PCT/EP2007/056353
(87) International publication number: WO 2008/015056

(56) References cited:
- EP-A- 0 482 444
- WO-A-01/40114
- WO-A-2004/014800

## Description

The invention relates to oxide particles comprising zinc and manganese, and to their production and use.

Manganese oxide-doped zinc oxide pigments are known in the prior art. DE-A-4033417 discloses a process in which, for example, zinc oxide and manganese oxide are ground in the presence of formic acid to a paste which is calcined at temperatures of 700 to 1100°C. In the course of this, manganese oxide is incorporated into the crystal lattice in the form of MnO, and yellow- to red-coloured pigments are formed. This product can also be used as a colouring agent in cosmetic formulations, for example a lipstick.

WO01/40114 likewise discloses a manganese oxide-doped zinc oxide. It is obtained by calcining a solution comprising a manganese salt and a zinc salt, preferably at 650°C to 750°C. The product thus obtained is intended to be suitable as a UV protectant, primarily for sunscreens. However, in-house experiments have shown that such a product can be dispersed only poorly, has only a low UV absorption and is therefore unsuitable as a UV protectant.

WO2005/072695 postulates that manganese oxide-doped zinc oxides can also be obtained by means of flame pyrolysis. However, it is not disclosed at any point how the flame pyrolysis is to be performed and what product arises from it.

It was an object of the present invention to provide a powder based on zinc oxide and manganese oxide which does not have the disadvantages of the prior art. It should in particular have a high UV absorption and a low photocatalytic activity, as far as possible be transparent, and be processable readily into cosmetic formulations.

The invention provides oxide particles comprising zinc and manganese
- which have a ratio of Zn/Mn of 15 to 300 in atom%/atom%,
- whose proportion of Zn, Mn and O is at least 99% by weight based on the oxide particles,
- which are present in the form of aggregated primary particles and have a BET surface area of 5 to 60 m²/g, preferably 20 to 30 m²/g,
- manganese is present in and on the primary particles, and
- in which only the signals of zinc oxide are detectable in the x-ray diffractogram.

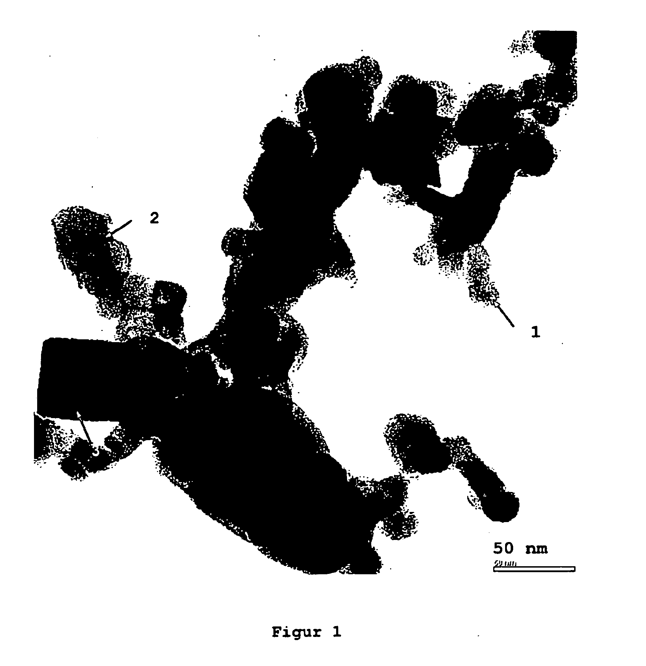

The inventive oxide particles are present in the form of aggregated primary particles. Figure 1 shows a transmission electron micrograph (transmission electron microcopy) in which the composition of individual regions has been determined in detail by means of EDX (energy-dispersive analysis of characteristic x-rays). The inventive oxide particles exhibit a very homogeneous distribution of zinc and manganese when the primary particles are compared with one another.

Moreover, it is characteristic of the inventive oxide particles that manganese is present in and on the primary particles. This can be confirmed, for example, by means of XPS-ESCA (XPS = x-ray photoelectron spectroscopy; ESCA = electron spectroscopy for chemical analysis). The evaluation of the XPS spectra is based on general recommendations according to DIN technical report No. 39, report DMA(A)97 of the National Physics Laboratory, Teddington, UK, and the knowledge to date regarding the development-accompanying standardization of the "Oberflächen- und Mikrobereichsanalysen" [surface and microregion analyses] working committee NMP816 (DIN).

In addition, the comparative spectra and corresponding results already present in the technical literature for the class present in each case are taken into account in the evaluation. After background subtraction, the values are calculated taking account of the relative sensitivity factors of the electron level specified in each case. Data are in atom per cent. The precision is ± 5%.

Advantageously, the inventive oxide particles may be present in a form in which the proportion of manganese on the surface of a primary particle is higher than in the remainder of the primary particle. This distribution can likewise be determined by XPS-ESCA analysis. To this end, the surface is initially ablated (sputtered) down to a depth of approx. 5 nm by bombardment with argon ions. According to the selection of the feedstocks and the process conditions, the inventive unsputtered oxide particles may have approx. 20 to 80% more Mn than the sputtered oxide particles.

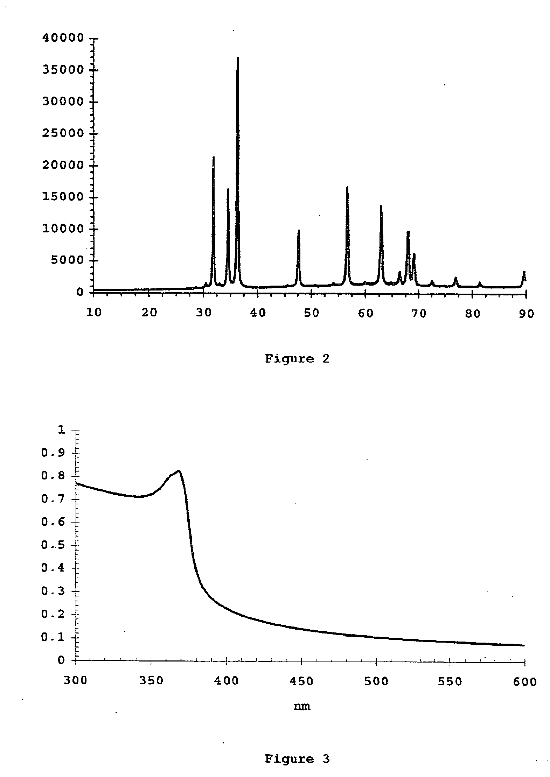

It is also characteristic of the inventive oxide particles that, in the x-ray diffractogram, only the signals of zinc oxide are detectable (see Figure 2).

The inventive oxide particles have a Zn/Mn ratio of 15 to 300, in atom%/atom%. This ratio is preferably 20 to 150. In this range, the inventive oxide particles have a particularly high UV absorption and a particularly low photocatalytic activity.

The proportion of Zn, Mn and O in the inventive oxide particles is at least 99% by weight in total. In general, the content is at least 99.5% by weight to 99.8% by weight.

If desired, selection of the suitable starting material and of the process allows the provision of oxide particles having a content of Zn, Mn and O of more than 99.8% by weight in total.

In the case that the inventive oxide particles are to be part of a cosmetic or pharmaceutical formulation, the proportion of Pb may be at most 20 ppm, that of As at most 3 ppm, that of Cd at most 15 ppm, that of Fe at most 200 ppm, that of Sb at most 1 ppm and that of mercury at most 1 ppm.

The inventive oxide particles may additionally have a carbon content of up to 1000 ppm, which may be caused by the use of carbon-containing feedstocks and/or the process. If required, selection of the feedstocks and/or the process can realise a carbon content of less than 250 ppm.

The inventive oxide particles advantageously have a photocatalytic activity, expressed by the photon efficiency and determined by the degradation of dichloroacetic acid (DCA) as a model harmful substance, which is less than 0.4%, more preferably less than 0.2%. It is also possible to provide inventive oxide particles in which no degradation of DCA whatsoever can be detected, i.e. which are photocatalytically inactive.

The degradation of the model harmful substance is monitored with reference to the consumption of sodium hydroxide solution to keep the pH constant. For the photocatalytic DCA degradation, the following stoichiometry is known: CHCl₂CO₂⁻ + O₂ → H⁺ + 2 Cl⁻ + 2 CO₂. For this purpose, first the degradation rate [nM/s] is determined from the initial slope of the proton formation curves, and, from this, the photon efficiency based on the incident light intensity (in %). The photon efficiency is an absolute measure of the photocatalytic activity. It is calculated from the degradation rate based on the photon flux.

The inventive oxide particles have good transparency. The transparency which, according to the invention, is defined as the maximum absorbance defined by the absorbance at 450 nm is preferably 2.5 to 8, and particular preference may be given to values of 3.5 to 7. The wavelength of maximum absorbance for the inventive oxide particles is 368 to 375 nm.

The inventive oxide particles may also be present in surface-modified form. In the context of the invention, surface-modified is understood to mean that at least some of the hydroxyl groups present on the surface of the oxide particles have reacted with a surface modifier to form a chemical bond. The chemical bond is preferably a covalent, ionic or coordinate bond between the surface modifier and the oxide particle, but also hydrogen bonds.

A coordinate bond is understood to mean a complex formation. For example, a Brønsted or Lewis acid/base reaction, a complex formation or an esterification can take place between the functional groups of the modifier and the oxide particles. The functional group which comprises the modifier preferably comprises carboxylic acid groups, acid chloride groups, ester groups, nitrile and isonitrile groups, OH groups, SH groups, epoxy groups, anhydride groups, acid amide groups, primary, secondary and tertiary amino groups, Si-OH groups, hydrolysable radicals of silanes or C-H-acidic moieties, as in beta-dicarbonyl compounds. The surface modifier may also comprise more than one such functional group, as, for example, in betaines, amino acids, EDTA.

Suitable surface modifiers may be: saturated or unsaturated mono- and polycarboxylic acids having 1 to 24 carbon atoms, for example formic acid, acetic acid, propionic acid, butyric acid, pentanoic acid, hexanoic acid, acrylic acid, methacrylic acid, crotonic acid, citric acid, adipic acid, succinic acid, glutaric acid, oxalic acid, maleic acid, fumaric acid, itaconic acid and stearic acid, and the corresponding acid anhydrides, acid chlorides, esters and amides, and also salts thereof, especially ammonium salts thereof. Also suitable are those carboxylic acids whose carbon chains are interrupted by O, S or NH groups, such as ether carboxylic acids (mono- and polyether carboxylic acids, and the corresponding acid anhydrides, acid chlorides, esters and amides), oxacarboxylic acids such as 3,6-dioxaheptanoic acid and 3,6,9-trioxadecanoic acid.

Mono- and polyamines of the general formula Q₃₋ₙNHₙ where n = 0, 1 or 2 and the Q radicals are each independently C₁-C₁₂-alkyl, especially C₁-C₆-alkyl and more preferably especially C₁-C₄-alkyl, for example methyl, ethyl, n-propyl and i-propyl and butyl, and also aryl, alkaryl or aralkyl having 6 to 24 carbon atoms, for example phenyl, naphthyl, tolyl and benzyl.

In addition, polyalkyleneamines of the general formula Y₂N(-Z-NY)_{y}-Y in which Y is independently Q or N, where Q is as defined above, y is an integer of 1 to 6, preferably 1 to 3, and Z is an alkylene group having 1 to 4, preferably 2 or 3 carbon atoms. Examples are methylamine, dimethylamine, trimethylamine, ethylamine, aniline, N-methylaniline, diphenylamine, triphenylamine, toluidine, ethylenediamine, diethylenetriamine.

Preferred beta-dicarbonyl compounds having 4 to 12, especially 5 to 8 carbon atoms, for example acetylacetone, 2,4-hexanedione, 3,5-heptanedione, acetoacetic acid, C₁-C₄-alkyl acetoacetate, such as ethyl acetoacetate, diacetyl and acetonylacetone.

Amino acids, such as beta-alanine, glycine, valine, aminocaproic acid, leucine and isoleucine.

Silanes which have at least one unhydrolysable group or a hydroxyl group, especially hydrolysable organosilanes which additionally have at least one unhydrolysable radical. Silanes of the general formula RₐSiX₄₋ₐ preferably serve as a surface-modifying reagent, in which the R radicals are the same or different and are each unhydrolysable groups, the X radicals are the same or different and are each hydrolysable groups or hydroxyl groups, and a is 1, 2 or 3. The a value is preferably 1.

In the general formula (I), the hydrolysable X groups, which may be the same or different from one another, are, for example, hydrogen or halogen (F, Cl, Br or I), alkoxy (preferably C₁-C₄-alkoxy, for example methoxy, ethoxy, n-propoxy, i-propoxy and butoxy), aryloxy (preferably C₆-C₁₀-aryloxy, for example phenoxy), acyloxy (preferably C₁-C₆-acyloxy, for example acetoxy or propionyloxy), alkylcarbonyl (preferably C₂-C₇-alkylcarbonyl, for example acetyl), amino, monoalkylamino or dialkylamino having preferably 1 to 12, in particular 1 to 6 carbon atoms. Preferred hydrolysable radicals are halogen, alkoxy groups and acyloxy groups. Particularly preferred hydrolysable radicals are C₁-C₄-alkoxy groups, especially methoxy and ethoxy.

The unhydrolysable R radicals, which may be the same or different from one another, may be unhydrolysable R radicals with or without a functional group.

The unhydrolysable R radical without a functional group is, for example, alkyl (preferably C₁-C₈-alkyl, such as methyl, ethyl, n-propyl, isopropyl, n-butyl, sec-butyl and tert-butyl, pentyl, hexyl, octyl or cyclohexyl), alkenyl (preferably C₂-C₆-alkenyl, for example vinyl, 1-propenyl, 2-propenyl and butenyl), alkynyl (preferably C₂-C₆-alkynyl, for example acetylenyl and propargyl), aryl (preferably C₆-C₁₀-aryl, for example phenyl and naphthyl), and corresponding alkaryls and aralkyls (for example tolyl, benzyl and phenethyl). The R and X radicals may optionally have one or more customary substituents, for example halogen or alkoxy. Preference is given to alkyltrialkoxysilanes. Examples are: CH₃SiCl₃, CH₃Si(OC₂H₅)₃, CH₃Si(OCH₃)₃, C₂H₅SiCl₃, C₂H₅Si(OC₂H₅)₃, C₂H₅Si (OCH₃)₃, C₃H₇Si (OC₂H₅)₃, (C₂H₅O)₃SiC₃H₆Cl, (CH₃)₂SiCl₂, (CH₃)₂Si(OC₂H₅)₂, (CH₃)₂Si(OH)₂, C₆H₅Si(OCH₃)₃, C₆H₅Si (OC₂H₅) ₃, C₆H₅CH₂CH₂Si (OCH₃)₃, (C₆H₅)₂SiCl₂, (C₆H₅)₂Si(OC₂H₅)₂, (i-C₃H₇)₃SiOH, CH₂=CHSi (OOCCH₃)₃, CH₂=CHSiCl₃, CH₂=CH-Si(OC₂H₅)₃, CH₂=CHSi(OC₂H₅)₃, CH₂=CH-Si(OC₂H₄OCH₃)₃, CH₂=CH-CH₂-Si (OC₂H₅)₃, CH₂=CH-CH₂-Si (OC₂H₅)₃, CH₂=CH-CH₂Si (OOOCH₃)₃, n-C₆H₁₃-CH₂-CH₂-Si (OC₂H₅)₃ and n-CH₁₇-CH₂CH₂-Si (OC₂H₅)₃.

The unhydrolysable R radical having a functional group may comprise, for example, as a functional group, an epoxy (for example glycidyl or glycidyloxy), hydroxyl, ether, amino, monoalkylamino, dialkylamino, optionally substituted anilino, amide, carboxyl, acryloyl, acryloyloxy, methacryloyl, methacryloyloxy, mercapto, cyano, alkoxy, isocyanato, aldehyde, alkylcarbonyl, acid anhydride and phosphoric acid group. These functional groups are bonded to the silicone atom via alkylene, alkenylene or arylene bridging groups which may be interrupted by oxygen or -NH- groups. The bridging groups contain preferably 1 to 18, preferably 1 to 8 and in particular 1 to 6 carbon atoms.

These divalent bridging groups and any substituents present, as in the case of the alkylamino groups, derive, for example, from the abovementioned monovalent alkyl, alkenyl, aryl, alkaryl or aralkyl radicals. Of course, the R radical may also have more than one functional group.

Preferred examples of unhydrolysable R radicals with functional groups are a glycidyl- or a glycidyloxy-(C₁-C₆)-alkylene radical, such as beta-glycidyloxyethyl, gamma-glycidyloxypropyl, delta-glycidyloxybutyl, epsilon-glycidyloxypentyl, omega-glycidyloxyhexyl and 2-(3,4-epoxycyclohexyl)ethyl, a (meth)acryloyloxy-(C₁-C₆)-alkylene radical, for example (meth)acryloyloxymethyl, (meth)acryloyloxyethyl, (meth)acryloyloxypropyl or (meth)acryloyloxybutyl, and a 3-isocyanatopropyl radical.

Examples of corresponding silanes are gamma-glycidyloxypropyltrimethoxysilane (GPTS), gamma-glycidyloxypropyltriethoxysilane (GPTES), 3-isocyanatopropyltriethoxysilane, 3-isocyanatopropyldimethylchlorosilane, 3-aminopropyltrimethoxysilane (APTS), 3-aminopropyltriethoxysilane (APTES), N-(2-aminoethyl)-3-aminopropyltrimethoxysilane, N-[N'-(2'-aminoethyl)-2-aminoethyl]-3-aminopropyltrimethoxysilane, hydroxylmethyltriethoxysilane, 2-[methoxy(polyethyleneoxy)-propyl]trimethoxysilane, bis(hydroxyethyl)-3-aminopropyltriethoxysilane, N-hydroxyethyl-N-methylaminopropyltriethoxysilane, 3-(meth)acryloyloxypropyltriethoxysilane and 3-(meth)acryloyloxypropyltrimethoxysilane.

The invention further provides a process for preparing the oxide particles comprising zinc and manganese, in which
- the solution of at least one oxidizable organic zinc compound and the solution of at least one oxidizable organic manganese compound, in each case in an organic solvent or solvent mixture, are atomized, separately or in the form of the combined solutions, in a Zn/Mn ratio of 15 to 300 in atom%/atom%, by means of an atomization gas to form an aerosol, and fed to a high-temperature zone of a reactor,
- in the high-temperature zone, the aerosol is reacted at temperatures of 500 to 2500°C with oxygen or an oxygenous gas, the amount of oxygen being at least sufficient to completely react the organic zinc compound and the organic manganese compound,
- the hot gases and the solid product are cooled and then the solid product is removed from the gases and
- the solid product is optionally reacted with a surface modifier.

In a preferred embodiment, the reaction temperature is generated by a flame obtained by igniting a mixture of a combustion gas and air, said flame burning into a reaction chamber and the aerosol being atomized into said flame, the amount of oxygen being at least sufficient to completely react the combustion gas, the organic zinc compound and the organic manganese compound.

The solutions comprising zinc and the manganese compounds may comprise identical or different organic solvents. When the solutions are combined, the stability of the resulting solution should be ensured. No opacity or precipitates should occur.

Suitable combustion gases may be hydrogen, methane, ethane, propane, natural gas, acetylene, carbon monoxide or mixtures of the aforementioned gases. Hydrogen is the most suitable. The temperature needed for the evaporation of the starting materials can be provided by a suitable selection of the aforementioned gases and the oxygen content of the flame. Preference is given to using hydrogen or mixtures with hydrogen.

It may also be advantageous to additionally introduce air (secondary air) into the reaction chamber.

Particular preference is given to an embodiment in which 1 < lambda ≤ 5. Most preferably, 1.5 ≤ lambda ≤ 2. The lambda value is defined as the quotient of the oxygen content of the oxygen-containing gas divided by the oxygen required for the complete oxidation of the combustion gas, of the organic zinc and manganese compounds, in each case in mol/h.

The mean residence time of the substances involved in the reaction in the reaction chamber can vary over a wide range. In general, it is 5 ms to 30 s.

Salts of carboxylic acids may preferably be used as the organic zinc compound and organic manganese compound. The term "carboxylic acids" encompasses aliphatic monocarboxylic acids, aliphatic dicarboxylic acids and aliphatic hydroxycarboxylic acids. Examples include the salts of formic acid, acetic acid, propionic acid, butyric acid, valeric acid, caproic acid, heptanoic acid, octanoic acid, 2-ethylhexanoic acid, pelargonic acid, decanoic acid, lauric acid, myristic acid, palmitic acid, stearic acid, arachic acid, citric acid, tartaric acid, lactic acid, gluconic acid and/or oxalic acid.

Owing to the availability and the economic viability, the salts of 2-ethylhexanoic acid have been found to be particularly advantageous.

The concentration of the organic zinc compound and of the organic manganese compound is, in total, in the combined solution preferably 5 to 30% by weight and more preferably 5 to 15% by weight, calculated as Zn and as Mn respectively.

The organic solvents used may advantageously, if appropriate by heating above the melting point, be one or more C₃-C₁₈ monocarboxylic acids, preferably C₅-C₁₆ monocarboxylic acids, more preferably C₇-C₁₄ monocarboxylic acids, or mixtures thereof with formic acid, acetic acid, alkanes and/or aromatics, such as C₆-C₈-alkanes, petroleum, benzene or toluene.

In order to achieve good solubility and stability of the organic zinc and manganese compounds, it has often been found to be advantageous to use mixtures of C₇-C₁₄-monocarboxylic acids with C₆-C₈-alkanes, petroleum, benzene and/or toluene.

In general, the proportion of C₇-C₁₄-monocarboxylic acids in these mixtures is 30 to 80% by weight, and proportions of less than 60% by weight may be particularly advantageous.

When the oxide particles are later to be part of a cosmetic or pharmaceutical formulation, particularly the purity of all feedstocks should be ensured. In particular, the organic zinc and manganese compounds should have at most 20 ppm of Pb, at most 3 ppm of As, at most 15 ppm of Cd, at most 200 ppm of Fe, at most 1 ppm of Sb and at most 1 ppm of Hg.

Oxide particles with the best properties can be obtained when the solution to be sprayed comprises a C₆-C₈ zinc carboxylate, a C₆-C₈ manganese carboxylate and a C₆-C₈-monocarboxylic acid, where the content of the C₆-C₈-monocarboxylic acid is 40 to < 60% by weight based on the solution.

The invention further provides a dispersion comprising the inventive particles.

The liquid phase of the dispersion may be water, one or more organic solvents or an aqueous/organic combination, the phases being miscible.

Liquid organic phases may in particular be methanol, ethanol, n-propanol and i-propanol, butanol, octanol, cyclohexanol, acetone, butanone, cyclohexanone, ethyl acetate, glycol ester, diethyl ether, dibutyl ether, anisole, dioxane, tetrahydrofuran, mono-, di-, tri- and polyglycol ether, ethylene glycol, diethylene glycol, propylene glycol, dimethylacetamide, dimethylformamide, pyridine, N-methylpyrrolidine, acetonitrile, sulpholane, dimethyl sulphoxide, nitrobenzene, dichloromethane, chloroform, carbon tetrachloride, ethylene chloride, pentane, hexane, heptane and octane, cyclohexane, petroleum, petroleum ether, methylcyclohexane, decalin, benzene, toluene and xylenes. Particularly preferred organic liquid phases are ethanol, n- and i-propanol, ethylene glycol, hexane, heptane, toluene and o-, m- and p-xylene.

Particularly water is preferred as the liquid phase.

The inventive dispersion may further comprise pH regulators, surface-active additives and/or preservatives.

The content of inventive oxide particles may preferably be 0.5 to 60% by weight. Particular preference is given to an aqueous dispersion containing 20 to 50% by weight, in particular 35 to 45% by weight, of the inventive oxide particles.

The pH of an inventive aqueous dispersion is preferably in a range of 6 to 9.

The mean particle size in the dispersion can be varied within a wide range using appropriate dispersion units. These may, for example, be rotor-stator machines, high-energy mills in which the particles are ground by collision with one another, planetary kneaders, stirred ball mills, ball mills which work as an agitating unit, agitated plates, ultrasound units or combinations of the aforementioned units.

A particularly small particle size can be obtained by using rotor-stator machines and high-energy mills. The mean particle size d₅₀ may assume values of less than 180 nm, in particular less than 140 nm, determined by means of dynamic light scattering.

The invention further provides a coating formulation which comprises the inventive oxide particles or the inventive dispersion and at least one binder.

Suitable binders may be polyacrylates, polyurethanes, polyalkyds, polyepoxides, polysiloxanes, polyacrylonitriles and/or polyesters. In the case of dispersions which have one or more reactive diluents as the liquid phase, a particularly suitable binder may be an aliphatic urethane acrylate, for example Laromer^{®} LR8987, BASF. The inventive coating formulation may more preferably comprise polyacrylates and/or polyurethanes.

The proportion of the binder in the coating formulation is preferably between 0.1 and 50% by weight. Particular preference is given to a range between 1 and 10% by weight.

The proportion of oxide particles in the coating formulation is preferably between 0.1 and 60% by weight. Particular preference is given to a range between 1 and 10% by weight.

In addition, the coating formulation, during the application, may comprise compounds for changing the rheology of the coating formulation. Particularly advantageous substances are fillers comprising silicon dioxide, particular preference being given to pyrogenic silicon dioxide. The amount may preferably be between 0.1 and 20% by weight, based on the overall coating formulation.

In addition, the coating formulation may comprise organic solvents such as ethanol, butyl acetate, ethyl acetate, acetone, butanol, THF, alkanes or mixtures of two or more of these substances in amounts of 1% by weight to 98% by weight, based on the overall coating formulation.

The inventive coating formulation may be used to coat substrates of wood, PVC, plastic, steel, aluminium, zinc, copper, MDF, glass, concrete.

The invention further provides a sunscreen formulation which comprises the inventive oxide particles.

The inventive oxide particles are present in the sunscreen formulation generally with a content of 0.5 to 20 by weight, preferably 1 to 10% by weight and more preferably 3 to 8% by weight.

Useful chemical UV filters include all water- or oil- soluble UV-A and UV-B filters known to those skilled in the art. For example, the inventive sunscreen formulation may comprise:
- paraaminobenzoic acid (PABA) and derivatives thereof, such as dimethyl-, ethyldihydroxypropyl-, ethylhexyldimethyl-, ethyl-, glyceryl- and 4-bis-(polyethoxy)-PABA.
- cinnamic esters, such as methylcinnamic esters and methoxycinnamic esters including octyl methoxycinnamate, ethyl methoxycinnamate, 2-ethylhexyl p-methoxycinnamate, isoamyl p-methoxycinnamate, diisopropyl cinnamate, 2-ethoxyethyl 4-methoxycinnamate, DEA methoxycinnamate (diethanolamine salt of p-methoxyhydroxycinnamate), diisopropyl methylcinnamate.
- benzophenones, such as 2,4-dihydroxy-, 2-hydroxy-4-methoxy-, 2,2'-dihydroxy-4,4'-dimethoxy-, 2,2'-dihydroxy-4-methoxy-, 2,2',4,4'-tetrahydroxy-, 2-hydroxy-4-methoxy-4'-methylbenzophenones, sodium 2,2'-dihydroxy-4,4'-dimethoxy-5-sulphobenzophenones.
- dibenzoylmethanes, such as butylmethoxydibenzoylmethane, in particular 4-tert-butyl-4'-methoxydibenzoylmethane.
- 2-phenylbenzimidazole-5-sulphonic acid and phenyldibenzimidazolesulphonic esters and salts thereof.
- diphenylacrylates, such as alkyl alpha-cyano-beta,beta-diphenylacrylates, such as octocrylene.
- triazines, such as 2,4,6-trianilino-(p-carbo-2-ethylhexyl-1-oxy)-1,3,5-triazine, ethylhexyl triazone and diethylhexylbutamidotriazone.
- camphor derivatives, such as 4-methylbenzylidene- and 3-benzylidenecamphor and terephthalylidenedicamphorsulphonic acid, benzylidenecamphorsulphonic acid, camphorbenzalkonium methosulphate and polyacrylamidomethylbenzylidenecamphor.
- salicylates, such as dipropylene glycol salicylate, ethylene glycol salicylate, ethylhexyl salicylate, isopropylbenzyl salicylate, methyl salicylate, phenyl salicylate, 3,3,5-trimethyl salicylate and TEA salicylate (compound of 2-hydroxybenzoic acid and 2,2',2"-nitrilotrisethanol).
- esters of 2-aminobenzoic acid.

The sunscreen formulation may further comprise compounds known to those skilled in the art, such as organic solvents, thickeners, emulsifiers, emollients, antifoams, antioxidants, plant extracts, moisturizing agents, perfumes, preservatives and/or dyes, complexing agents, anionic, cationic, nonionic or amphoteric polymers or mixtures thereof, propellant gases and finely divided powders, including metal oxide pigments with a particle size of 100 nm to 20 µm.

Suitable emollients are in particular avocado oil, cotton seed oil, behenyl alcohol, butyl myristate, butyl stearate, cetyl alcohol, cetyl palmitate, decyl oleate, dimethyl polysiloxane, di-n-butyl sebacate, thistle oil, eicosanyl alcohol, glyceryl monoricinoleate, hexyl laurate, isobutyl palmitate, isocetyl alcohol, isocetyl stearate, isopropyl isostearate, isopropyl laurate, isopropyl linoleate, isopropyl myristate, isopropyl palmitate, isopropyl stearate, isostearic acid, cocoa butter, coconut oil, lanolin, lauryl lactate, corn oil, myristyl lactate, myristyl myristate, evening primrose oil, octadecan-2-ol, olive oil, palmitic acid, palm kernel oil, polyethylene glycol, rapeseed oil, castor oil, sesame oil, soya oil, sunflower oil, stearic acid, stearyl alcohol, triethylene glycol.

Suitable emulsifiers are in particular glycerol monolaurate, glycerol monooleate, glycerol monostearate, PEG 1000 dilaurate, PEG 1500 dioleate, PEG 200 dilaurate, PEG 200 monostearate, PEG 300 monooleate, PEG 400 dioleate, PEG 400 monooleate, PEG 400 monostearate, PEG 4000 monostearate, PEG 600 monooleate, polyoxyethylene(4) sorbitol monostearate, polyoxyethylene (10) cetyl ether, polyoxyethylene(10) monooleate, polyoxyethylene(10) stearyl ether, polyoxyethylene(12) lauryl ether, polyoxyethylene(14) laurate, polyoxyethylene(2) stearyl ether, polyoxyethylene(20) cetyl ether, polyoxyethylene(20) sorbitol monolaurate, polyoxyethylene(20) sorbitol monooleate, polyoxyethylene(20) sorbitol monopalmitate, polyoxyethylene(20) sorbitol monostearate, polyoxyethylene(20) sorbitol trioleate, polyoxyethylene(20) sorbitol tristearate, polyoxyethylene(20) stearyl ether, polyoxyethylene(23) lauryl ether, polyoxyethylene(25) oxypropylene monostearate, polyoxyethylene(3.5) nonylphenol, polyoxyethylene(4) lauryl ether, polyoxyethylene(4) sorbitol monolaurate, polyoxyethylene(5) monostearate, polyoxyethylene(5) sorbitol monooleate, polyoxyethylene(50) monostearate, polyoxyethylene(8) monostearate, polyoxyethylene(9.3) octylphenol, polyoxyethylene sorbitol lanolin derivatives, sorbitol monolaurate, sorbitol monooleate, sorbitol monopalmitate, sorbitol monostearate, sorbitol sesquioleate, sorbitol tristearate, sorbitol trioleate.

Suitable propellant gases may be propane, butane, isobutane, dimethyl ether and/or carbon dioxide.

Suitable finely divided powders may be chalk, talc, kaolin, colloidal silicon dioxide, sodium polyacrylate, tetraalkyl- and/or trialkylarylammonium smectites, magnesium aluminium silicates, montmorillonite, aluminium silicates, fumed silicon dioxide, fumed titanium dioxide.

Typically, the inventive sunscreen may be in the form of an emulsion (O/W, W/O or multiple), aqueous or aqueous-alcoholic gel or oil gel, and be supplied in the form of lotions, creams, milk sprays, mousse, as stick or in other customary forms.

### Examples

### Solutions used:

Solution A:
   Zinc octoate: 10% by weight, calculated as Zn
   Petroleum spirit: 42% by weight
   C₆-C₁₉ Fatty acids: 46% by weight
   2-(2-Butoxyethoxyethanol): 2% by weight
Solution B:
   Zinc octoate: 22% by weight, calculated as Zn
   C₆-C₁₉ Fatty acids: 78% by weight
Solution C:
   Manganese octoate: 10% by weight, calculated as Mn
   Naphtha: 34.5
   C₆-C₁₉ Fatty acids: 55.5

### Example 1:

1500 g/h of solution A and 40 g/h of solution C are combined. The combined solutions are atomized into a reaction chamber to form an aerosol by means of atomization air (2 m³ (STP)/h) in a two-substance nozzle. The mean droplet diameter D₃₀ is less than 100 µm. Here, a hydrogen/oxygen flame composed of hydrogen (1.5 m³ (STP) /h) and air (23 m³ (STP) /h) burns, in which the aerosol is reacted. The temperature 300 mm below the flame is 980°C. Subsequently, the reaction mixture is cooled and the solid product is removed from gaseous substances on a filter.

Inventive examples 2 and 3 are performed analogously to Example 1.

The feedstocks and reaction conditions of Examples 1 to 3 and characteristic values of the resulting oxide particles are reproduced in Table 1.

Figure 1 shows a transmission electron micrograph of the inventive oxide particles from Example 1.

Table 2 shows the results of the determination of the particle composition of the oxide particles from Example 1 as specified in the transmission electron micrograph in Figure 1, by means of high-resolution TEM in combination with EDX. The bulk analysis of this powder showed 2.8% Mn. The homogeneous composition of the particles is clearly evident.

**Table 1: Feedstocks and reaction conditions; analytical values of the oxide particles**

| **Example** | | **1** | **2** | **3** |
|---|---|---|---|---|
| Solution A | g/h | 1500 | 920 | - |
| Solution B | g/h | - | - | 630 |
| Solution C | g/h | 60 | 40 | 50 |
| Atomization air | m³ (STP) /h | 2 | 3 | 4 |
| Hydrogen | m³ (STP) /h | 1.5 | 1.5 | 1.5 |
| Air | m³ (STP) /h | 23 | 19 | 21 |
| Lambda | | 1.39 | 1.93 | 1.45 |
| Temperature | °C | 980 | 750 | 1010 |
| BET surface area | m²/g | 25 | 28 | 21 |
| Zn/Mn | | 25 | 22 | 28.6 |
| Zn + Mn + O | % by wt. | > 99.7 | > 99.7 | > 99.7 |

**Table 2:Particle composition (Example 1)**

| **Element** | **Particle 1** | **Particle 2** | **Particle 3** |
|---|---|---|---|
| Zn | 72.53 ± 3.79 | 72.57 ± 2.54 | 72.94 ± 2.03 |
| Mn | 2.91 ± 0.32 | 3.17 ± 0.34 | 3.24 ± 0.28 |
| O | 24.56 ± 1.89 | 24.26 ± 1.21 | 23.82 ± 1.09 |
| Zn/Mn | 24.9 | 22.9 | 22.5 |

**Table 3:Quantitative XPS/ESCA analysis**

| **Ex.** | | **Mn2p (atom%)** | **Zn2p_{3/2} (atom%)** | **Mn sputtered/ unsputtered**** |
|---|---|---|---|---|
| 1 | unsputtered | 2.21 | 49.8 | 82.6 |
| | sputtered* | 1.21 | 59.2 | |
| 2 | unsputtered | 1.13 | 38.7 | 22.8 |
| | sputtered* | 0.92 | 46.5 | |

| | | | | |
|---|---|---|---|---|
| * Sputtered: after ablation of the surface by bombardment with argon ions; **) 100 x (Mn sputtered - Mn unsputtered)/Mn sputtered | | | | |

Figure 2 shows an x-ray diffractogram of the inventive oxide particles from Example 1 with 2 theta (in °) as the x axis and "counts" as the y axis. Only the signals of zinc oxide are detectable.

Table 3 shows the results of the quantitative XPS-ESCA analysis of unsputtered and sputtered material from Examples 1 and 2. It is clearly evident that Mn is present enriched at the surface.

Photocatalytic activity of the oxide particles from Example 1 determined by the degradation of dichloroacetic acid (DCA): over a period of 6 hours, no degradation of DCA is detectable in a reproducible manner. The oxide powder from Example 1 which forms the basis of the dispersion has no photocatalytic activity.

Figure 3 shows the UV spectrum of the oxide particles from Example 1 (conditions: 0.05% by weight, layer thickness: 1 mm).
The absorbance is plotted here against the wavelength in nm. It can be seen that the inventive particles bring about a high UV absorption and at the same time have high transparency. The transparency is, expressed as the absorbance at 368 nm divided by absorbance at 450 nm, 5.86.

### Example 4: Preparation of an inventive dispersion

The oxide, particles from Example 1 are added with stirring in portions to 50 g of water to which 0.1% by weight of polyacrylic acid in the form of the sodium salt has been added, until a solids content of 10% by weight results. Subsequently, dispersion is effected for one minute in each case with an ultrasound finger (diameter: 7 mm, instrument: Dr. Hielscher UP 400s ultrasound processor, power: 400 W).

### Example 5: Preparation of an inventive coating formulation based on acrylate/polyurethane

The dispersion from Example 4 is added under dispersing conditions to a commercial acrylate/polyurethane binder formulation (Relius Aqua Siegel Gloss), so as to result in a coating formulation having a proportion of composite particles of 2% by weight.

### Example 6: Preparation of an inventive coating formulation based on acrylate

Procedure as in Example 5, except using a commercial acrylate binder formulation (Macrynal SM 510 (Cytec), Desmodur N75 (Bayer)).

### Example 7: UV stability in coating of wood

The coating formulations from Example 5 and 6 are used to coat in each case 3 pinewood samples which had each been pretreated with a primer (Relius Aqua Holz Grund) (QUV-B 313; DIN EN 927-6, ISO 11507, ASTM D 4857). The comparison used was pinewood samples which had been coated with a coating formulation which is free of composite particles and is based on acrylate/polyurethane (Relius Aqua Siegel Gloss).

After a test time of 1000 hours, the coatings from Examples 5 and 6, in contrast to the coating without composite particles, exhibit significantly lower yellowing, significantly higher gloss and no embrittlement or cracks in the coating.

### Example 8: Preparation of an inventive sunscreen formulation

The following formulation was used to prepare a sunscreen with 4% by weight of the inventive oxide particles according to Example 1.

| **Phase** | **Constituent** | **% by wt.** |
|---|---|---|
| A | Isolan GI 34 | 3.0 |
| | Castor oil | 1.2 |
| | Tegesoft OP | 10.0 |
| | Tegesoft Liquid | 5.0 |
| | 86% Glycerol | 3.0 |
| B | Paracera W80 | 1.8 |
| | Isohexadecane | 5.0 |
| C | Composite particles according to Example 1 | 4.0 |
| D | Magnesium sulphate | 0.5 |
| | Demineralized water | 66.5 |

Phase A is heated to 70°C in a mixer. After melting on a magnetic hotplate at 80°C, phase B is added to phase A. Phase C is stirred into the oil phase at approx. 300 rpm and under vacuum. Phase D is likewise heated to 70°C and added to the mixture of A-C under vacuum.

## Claims

1. Oxide particles comprising zinc and manganese,
**characterized in that**
- they have a ratio of Zn/Mn of 15 to 300 in atom%/atom%,
- the proportion of Zn, Mn and O is at least 99% by weight based on the oxide particles,
- they are present in the form of aggregated primary particles and have a BET surface area of 10 to 60 m²/g,
- manganese is present in and on the primary particles,
- only the signals of zinc oxide are detectable in the x-ray diffractogram.

2. Oxide particles comprising zinc and manganese according to Claim 1, **characterized in that** the proportion of manganese on the surface of a primary particle is higher than in the remainder of the primary particle.

3. Oxide particles according to Claim 1 or 2, **characterized in that** they are present in surface-modified form.

4. Process for preparing the oxide particles comprising zinc and manganese according to Claims 1 to 3, **characterized in that**
- the solution of at least one oxidizable organic zinc compound and the solution of at least one oxidizable organic manganese compound, in each case in an organic solvent or solvent mixture, are atomized, separately or in the form of the combined solutions, in a Zn/Mn ratio of 15 to 300 in atom%/atom%, by means of an atomization gas to form an aerosol, and fed to a high-temperature zone of a reactor,
- in the high-temperature zone, the aerosol is reacted at temperatures of 500 to 2500°C with oxygen or an oxygenous gas, the amount of oxygen being at least sufficient to completely react the organic zinc compound and the organic manganese compound,
- the hot gases and the solid product are cooled and then the solid product is removed from the gases and
- the solid product is optionally reacted with a surface modifier.

5. Process according to Claim 4, **characterized in that** the reaction temperature is generated by a flame obtained by igniting a mixture of a hydrogenous combustion gas and air, said flame burning into a reaction chamber and the aerosol being atomized into said flame, the amount of oxygen being at least sufficient to completely react the hydrogenous combustion gas, the organic zinc compound and the organic manganese compound.

6. Process according to Claims 4 and 5, **characterized in that** the mean residence time of the substances involved in the reaction is 5 ms to 30 s.

7. Process according to Claims 4 to 6, **characterized in that** the organic zinc compound and organic manganese compound used are in each case salts of carboxylic acids.

8. Process according to Claims 4 to 7, **characterized in that** the concentration of the organic zinc compound and of the organic manganese compound in the solution to be sprayed is 5 to 30% by weight in total.

9. Process according to Claims 4 to 8, **characterized in that** the organic solvent used comprises one or more C₃-C₁₈ monocarboxylic acids or mixtures thereof with formic acid, acetic acid, C₆-C₈-alkanes, petroleum, benzene and/or toluene.

10. Process according to Claims 4 to 9, **characterized in that** the solution to be sprayed comprises a C₆-C₈-zinc carboxylate and a C₆-C₈-manganese carboxylate with a C₆-C₈-monocarboxylic acid, where the content of the C₆-C₈-monocarboxylic acid is 40 to < 60% by weight based on the solution.

11. Dispersion comprising the oxide particles comprising zinc and manganese according to Claims 1 to 3.

12. Coating composition comprising the oxide particles comprising zinc and manganese according to Claims 1 to 3 or the dispersion according to Claim 11 and at least one binder.

13. Sunscreen formulation comprising the oxide particles comprising zinc and manganese according to Claims 1 to 3 or the dispersion according to Claim 11.

## Patentansprüche

1. Zink und Mangan enthaltende Oxidpartikel, **dadurch gekennzeichnet, dass**
- sie ein Verhältnis Zn/Mn von 15 bis 300, in Atom-%/Atom-%, aufweisen,
- der Anteil von Zn, Mn und O wenigstens 99 Gew.-%, bezogen auf die Oxidpartikel, beträgt,
- sie in Form von aggregierten Primärpartikeln vorliegen und eine BET-Oberfläche von 10 bis 60 m²/g aufweisen,
- Mangan in und auf den Primärpartikeln vorliegt,
- im Röntgendiffraktogramm nur die Signale von Zinkoxid detektierbar sind.

2. Zink und Mangan enthaltende Oxidpartikel nach Anspruch 1, **dadurch gekennzeichnet, dass** der Anteil an Mangan auf der Oberfläche eines Primärpartikels höher ist als im Rest des Primärpartikels.

3. Oxidpartikel nach Anspruch 1 oder 2, **dadurch gekennzeichnet, dass** sie in oberflächenmodifizierter Form vorliegen.

4. Verfahren zur Herstellung der Zink und Mangan enthaltenden Oxidpartikel gemäss den Ansprüchen 1 bis 3, **dadurch gekennzeichnet, dass** man
- die Lösung mindestens einer oxidierbaren organischen Zinkverbindung und die Lösung mindestens einer oxidierbaren organischen Manganverbindung, jeweils in einem organischen Lösungsmittel oder Lösungsmittelgemisch, getrennt oder in Form der vereinigten Lösungen, in einem Verhältnis Zn/Mn von 15 bis 300, in Atom-%/Atom-%, mittels eines Verdüsungsgases unter Bildung eines Aerosoles zerstäubt und einer Hochtemperaturzone eines Reaktors zuführt,
- in der Hochtemperaturzone das Aerosol bei Temperaturen von 500 bis 2500°C mit Sauerstoff oder einem sauerstoffhaltigen Gas zur Reaktion bringt, wobei die Menge an Sauerstoff wenigstens ausreicht, um die organische Zinkverbindung und die organische Manganverbindung vollständig umzusetzen,
- die heissen Gase und das feste Produkt kühlt und anschliessend das feste Produkt von den Gasen abtrennt und
- das feste Produkt gegebenenfalls mit einem Oberflächenmodifizierungsmittel umsetzt.

5. Verfahren nach Anspruch 4, **dadurch gekennzeichnet, dass** die Reaktionstemperatur durch eine durch die Zündung eines Gemisches eines wasserstoffhaltigen Brenngases und Luft erhaltene Flamme erzeugt wird, die in einen Reaktionsraum hinein verbrennt und in die das Aerosol zerstäubt wird, wobei die Menge an Sauerstoff wenigstens ausreicht, um das wasserstoffhaltige Brenngas, die organische Zinkverbindung und die organische Manganverbindung vollständig umzusetzen.

6. Verfahren nach den Ansprüchen 4 und 5, **dadurch gekennzeichnet, dass** die mittlere Verweilzeit der an der Reaktion beteiligten Stoffe 5 ms bis 30 s ist.

7. Verfahren nach den Ansprüchen 4 bis 6, **dadurch gekennzeichnet, dass** als organische Zinkverbindung und organische Manganverbindung jeweils Salze von Carbonsäuren eingesetzt werden.

8. Verfahren nach den Ansprüchen 4 bis 7, **dadurch gekennzeichnet, dass** die Konzentration der organischen Zinkverbindung und der organischen Manganverbindung in der zu versprühenden Lösung in Summe 5 bis 30 Gew.-% beträgt.

9. Verfahren nach den Ansprüchen 4 bis 8, **dadurch gekennzeichnet, dass** als organisches Lösungsmittel eine oder mehrere C₃-C₁₈ Monocarbonsäuren oder deren Gemische mit Ameisensäure, Essigsäure, C₆-C₈-Alkanen, Benzin, Benzol und/oder Toluol eingesetzt werden.

10. Verfahren nach den Ansprüchen 4 bis 9, **dadurch gekennzeichnet, dass** die zu versprühende Lösung ein C₆-C₈-Zinkcarboxylat und ein C₆-C₈-Mangancarboxylat mit einer C₆-C₈-Monocarbonsäure enthält, wobei der Anteil der C₆-C₈-Monocarbonsäure 40 bis < 60 Ges.-%, bezogen auf die Lösung, ist.

11. Dispersion enthaltend die Zink und Mangan enthaltenden Oxidpartikel gemäss den Ansprüchen 1 bis 3.

12. Beschichtungszusammensetzung enthaltend die Zink und Mangan enthaltenden Oxidpartikel gemäss den Ansprüchen 1 bis 3 oder die Dispersion gemäss dem Anspruch 11 und wenigstens ein Bindemittel.

13. Sonnenschutzformulierung enthaltend die Zink und Mangan enthaltenden Oxidpartikel gemäss den Ansprüchen 1 bis 3 oder die Dispersion gemäss dem Anspruch 11.

## Revendications

1. Particules d'oxyde comprenant du zinc et du manganèse, **caractérisées en ce que**
- elles ont un rapport Zn/Mn de 15 à 300 % atomique,
- la proportion d'atomes de Zn, de Mn et d'O est d'au moins 99 % en poids basée sur les particules d'oxyde,
- elles sont présentes sous la forme de particules primaires agrégées et elles possèdent une surface spécifique BET de 10 à 60 m²/g,
- le manganèse est présent dans et sur les particules primaires,
- seuls les signaux d'oxyde de zinc sont détectables sur le spectre de diffraction des rayons X.

2. Particules d'oxyde comprenant du zinc et du manganèse selon la revendication 1, **caractérisées en ce que** la proportion de manganèse sur la surface d'une particule primaire est supérieure à celle dans le reste de la particule primaire.

3. Particules d'oxyde selon la revendication 1 ou 2, **caractérisées en ce qu'**elles sont présentes dans une forme à surface modifiée.

4. Procédé de préparation des particules d'oxyde comprenant du zinc et du manganèse selon les revendications 1 à 3, **caractérisé en ce que**
- la solution d'au moins un composé de zinc organique oxydable et la solution d'au moins un composé de manganèse organique oxydable, dans chaque cas dans un solvant organique ou un mélange de solvants, sont atomisées, séparément ou sous la forme des solutions combinées, dans un rapport Zn/Mn de 15 à 300 % atomique, au moyen d'un gaz d'atomisation pour former un aérosol, et sont alimentées vers une zone à température élevée d'un réacteur,
- dans la zone à température élevée, on fait réagir l'aérosol à des températures de 500 à 2500 °C avec de l'oxygène ou un gaz contenant de l'oxygène, la quantité d'oxygène étant au moins suffisante pour faire réagir complètement le composé de zinc organique et le composé de manganèse organique,
- les gaz chauds et le produit solide sont refroidis et le produit solide est ensuite retiré des gaz et
- le produit solide réagit éventuellement avec un modificateur de surface.

5. Procédé selon la revendication 4, **caractérisé en ce que** la température de la réaction est générée par une flamme obtenue en enflammant un mélange d'air et de gaz de combustion contenant de l'hydrogène, ladite flamme brûlant dans une chambre de réaction et l'aérosol étant atomisé dans ladite flamme, la quantité d'oxygène étant au moins suffisante pour faire complètement réagir le gaz de combustion contenant de l'hydrogène, le composé de zinc organique et le composé de manganèse organique.

6. Procédé selon les revendications 4 et 5,
**caractérisé en ce que** le temps de séjour moyen des substances prenant part à la réaction est de 5 ms à 30 s.

7. Procédé selon les revendications 4 à 6,
**caractérisé en ce que** le composé de zinc organique et le composé de manganèse organique utilisés sont dans chaque cas des sels d'acides carboxyliques.

8. Procédé selon les revendications 4 à 7,
**caractérisé en ce que** la concentration du composé de zinc organique et du composé de manganèse organique dans la solution à vaporiser varie de 5 à 30 % en poids au total.

9. Procédé selon les revendications 4 à 8,
**caractérisé en ce que** le solvant organique utilisé comprend un ou plusieurs acides monocarboxyliques de C₃ à C₁₈ ou des mélanges de ceux-ci avec de l'acide formique, de l'acide acétique, des alcanes de C₆ à C₈, du pétrole, du benzène et/ou du toluène.

10. Procédé selon les revendications 4 à 9,
**caractérisé en ce que** la solution à vaporiser comprend un carboxylate de zinc de C₆ à C₈ et un carboxylate de manganèse de C₆ à C₈ avec un acide monocarboxylique de C₆ à C₈, dans lequel la teneur de l'acide monocarboxylique de C₆ à C₈ varie de 40 à moins de 60 % en poids basée sur la solution.

11. Dispersion comprenant les particules d'oxyde qui comprennent du zinc et du manganèse selon les revendications 1 à 3.

12. Composition de revêtement comprenant les particules d'oxyde qui comprennent du zinc et du manganèse selon les revendications 1 à 3 ou la dispersion selon la revendication 11 et au moins un liant.

13. Formulation d'écran solaire comprenant les particules d'oxyde comprenant du zinc et du manganèse selon les revendications 1 à 3 ou la dispersion selon la revendication 11.
